# EUROPEAN PATENT APPLICATION

(11) **EP 2 204 230 A1**
(43) Date of publication of application: **07.07.2010**
(21) Application number: 08840607.9
(22) Date of filing: 16.10.2008
(51) Int. Cl.: B01J 2/04

(54) **METHOD FOR PREPARATION OF PARTICLES**

(30) Priority: 17.10.2007 ES 200702724
(71) Applicant: Activery Biotech, Sl, 31003 Pamplona (Navarra) (ES)
(72) Inventor: KORDIKOWSKI, Andreas, E-08195 Sant Cugat Del Vallés (Barcelona) (ES); SAEZ MUÑOZ, Cristina, E-08320 El Masnou (Barcelona) (ES)
(74) Representative: Gislon, Gabriele
(86) International application number: PCT/ES2008/000643
(87) International publication number: WO 2009/050315

(57) **Abstract**

The present invention relates to a new method for preparing micron-sized particles which comprises introducing in the particle formation vessel a solute solution in a solvent and a compressed fluid under supercritical conditions, wherein the vessel in which the particles are formed is at a pressure comprised between atmospheric pressure and 15 bar. The particles obtained present a small size, a limited size distribution, and low agglomeration. Particles with suitable fluidity can furthermore be prepared from products, for example, pharmaceutical active ingredients and/or excipients, with a low glass transition temperature.

## Description

### Field of the Art

The present invention relates to a new method for preparing micron-sized particles in which a compressed fluid under supercritical conditions is used.

### Prior State of the Art

The technology of fluids under supercritical conditions has been used for decades in many fields of the art, such as in the food industry, in the manufacture of pigments and polymers, and in the pharmaceutical industry, for example.

In this last field, it has been widely used for natural active ingredient extraction, in product sterilization, and in preparing active ingredient particles, alone or combined with excipients.

The article by M. Perrut et al., Particle design using supercritical fluids: Literature and patent survey, J. Sup. Fluids, 2001, 20, 179 - 219, describes different methods developed for preparing particles in which fluids under supercritical conditions are used.

Despite the existence of conventional processes for preparing small particles, for example milling, these processes based on supercritical fluids have received considerable attention because they generally allow obtaining homogenous micron-sized or sub-micron-sized particles with suitable control of the size and of the morphology of the powder, and furthermore the obtained powder is solvent-free.

The RESS (Rapid Expansion of Supercritical Solutions) process consists of dissolving the product in the supercritical fluid and quickly depressurizing the solution through a suitable nozzle, causing an extremely rapid nucleation of the product, and the acquisition of a highly disperse material. This process has been known for some time and is attractive because of the absence of organic solvent. Nevertheless, it only applies to non-polar compounds presenting reasonable solubility in the supercritical fluid, and furthermore the rapid expansion of the supercritical solution through the nozzle can cause the supercritical fluid to freeze and obstruct said nozzle.

The GAS or SAS (Gas or Supercritical fluid Anti-solvent) process consists of reducing the solvent power of a polar liquid solvent in which the substrate is dissolved by means of saturation with a fluid under supercritical conditions, for example carbon dioxide, which causes the recrystallization or precipitation of the substrate.

Depending on the desired morphology of the particles, this GAS process can be implemented in different manners:
- In the ASES (Aerosol Solvent Extraction System) process, a solution of a substrate or substrates in an organic solvent is sprayed in a vessel containing a fluid under supercritical conditions. Micro- or nanoparticles are obtained by means of this process.
- The SEDS (Solution Enhanced Dispersion by Supercritical fluids) process consists of the combined spraying of the substrate solution and of a supercritical fluid stream through suitable nozzles. An intimate mixture of the supercritical fluid and the solution, which leads to the formation of the particles, is thus obtained.

The crucial operation of the GAS process is the mixture of the solution and the supercritical fluid. To obtain a rapid and intimate mixture, the solution must be dispersed in the form of small droplets in the supercritical fluid. Different devices for injecting the solution and the supercritical fluid in a particle formation vessel for the purpose of obtaining a suitable mixture have been proposed in the state of the art.

In the PGSS (Particles from Gas-Saturated Solutions or Suspensions) process, a supercritical fluid is added to a mixture of substrate and molten polymer, and then the mixture is rapidly depressurized through a nozzle, whereby causing the formation of particles.

Patent application ES-A-2170008 describes the DELOS (Depressurization of an Expanded Liquid Organic Solution) process used for preparing finely divided solid particles of a substrate. In said process a homogenous solution of the substrate is prepared in a mixture of a solvent and carbon dioxide at a high temperature and pressure, which acts as a co-solvent. The particles are formed upon depressurizing the solution at atmospheric pressure due to the evaporation of the carbon dioxide and the subsequent abrupt cooling of the solution, which leads to a nucleation in the entire mass.

It has been observed that the DELOS process is not suitable for those substrates presenting insufficient solubility in the mixture of the solvent and supercritical fluid under the pressure and temperature conditions required to perform said process (for example a combination of nifedipine and polyvinylpyrrolidone). It has also been observed that the yield is typically insufficient because the recovery of the particles is hindered by the low temperatures reached during depressurization.

Patent application WO-A-02/068107 describes a method for preparing microparticles, particularly proteins, which is based on the GAS process, i.e., the anti-solvent properties of the supercritical fluid are used to cause the precipitation of the particles. Said process comprises separately introducing a solution of a substrate in a solvent and a supercritical fluid in a vessel which is at a pressure comprised between 80 and 120 bar and at a temperature comprised between 35°C and 50°C.

Patent application WO-A-03/008082 describes a modification of the SEDS process which comprises introducing, through separate conduits, a solution of the substrate in a solvent and a compressed anti-solvent fluid, into a particle formation vessel the pressure of which is greater than 74 bar.

It has been observed that the particles obtained with this modified SEDS process are strongly agglomerated and present a non-uniform size distribution. Furthermore, said process is not suitable for plasticizing substances, such as for example polyvinylpyrrolidone, which liquefies at moderately high pressures.

Therefore, it is necessary to have an improved process which overcomes the drawbacks of the processes described in the state of the art and allows preparing particles with a more homogenous size distribution and with reduced agglomeration, as well as preparing products with suitable fluidity from active ingredients and/or excipients with a low amorphous-state glass transition temperature which, under the conditions of the processes described in the state of the art, are plasticized products. Said active ingredients can include griseofulvin, ibuprofen, lovastatin and simvastatin, among others. The excipients could include: polyvinylpyrrolidone (PVP), polyethylene glycol, aspartame, maltitol, dipalmitoylphosphatidylcholine, distearoylphosphatidylcholine, or vitamin E among others.

### Object of the Invention

The authors of this invention have discovered a new method for preparing micron-sized particles which allows obtaining them with improved characteristics and is suitable for preparing particles with suitable fluidity from active ingredients and/or excipients with a low glass transition temperature.

The object of the invention is a method for preparing particles.

### Description of the Drawings

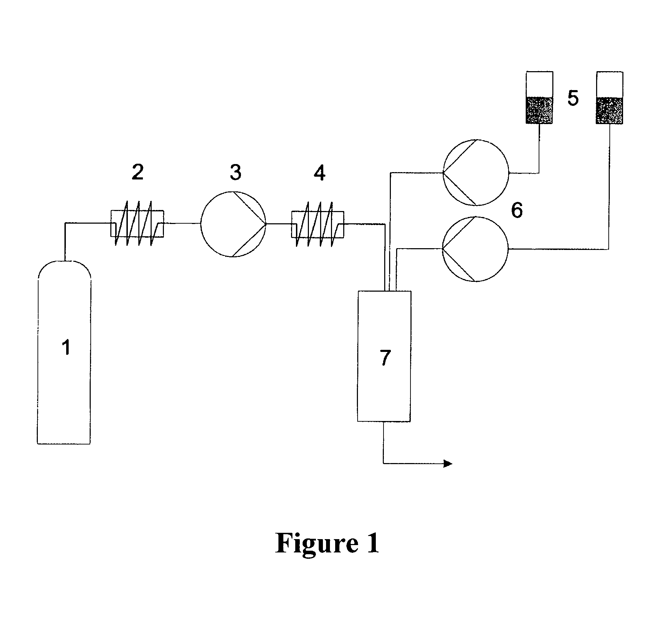

Figure 1 shows a schematic diagram of a piece of equipment suitable for carrying out the method of invention, which includes the following technical elements:
   - (1) vessel containing the compressed fluid in liquid state.
   - (2) heat exchanger for cooling the compressed fluid.
   - (3) high-pressure pump.
   - (4) heat exchanger for heating the compressed fluid.
   - (5) vessels in which the solute solution is located.
   - (6) injection pumps of the same type as those used in HPLC.
   - (7) particle formation vessel.
Figure 2 shows a scanning electron microscopy (SEM) image of the paracetamol particles prepared according to the method of the invention in Example 1.
Figure 3 shows an SEM image of the paracetamol particles prepared according to the method of the invention in Example 2.
Figure 4 shows an SEM image of the paracetamol particles prepared in comparative Example 1 according to the method described in patent application WO-A-03/008082.
Figure 5 shows an SEM image of the nifedipine and polyvinylpyrrolidone particles obtained in Example 3 according to the method of the invention.

### Detailed Description of the Invention

The method for preparing particles of the invention comprises introducing in the particle formation vessel through separate conduits:
1) a flow of a compressed fluid under supercritical conditions, and
2) a flow of a solute solution in a solvent,
   and collecting the particles formed,
   wherein:
   i. the inlet pressure of the compressed fluid into the particle formation vessel is comprised between 1.01 and 9 times the critical pressure thereof expressed in bars,
   ii. the inlet temperature of the compressed fluid into the particle formation vessel is comprised between 1.01 and 4 times the critical temperature thereof expressed in degrees Kelvin,
   iii. the angle formed by the axes of each of the flows is comprised between 0° and 180°,
   iv. the solute solution inlet takes place within the central line of the compressed fluid jet,
   v. the temperature in the particle formation vessel is comprised between 0.8 and 1.25 times the critical temperature of the compressed fluid expressed in degrees Kelvin,
   **characterized in that** the particle formation vessel is at a pressure comprised between atmospheric pressure and 15 bar.

In the method of the invention, a solute solution in a solvent and a compressed fluid under supercritical conditions are introduced, through separate conduits, in a vessel which is at a moderate pressure and under operating conditions suitable for obtaining particles of the solute.

### Compressed Fluid

In the method of the invention, the compressed fluid is under supercritical conditions at the inlet point into the particle formation vessel.

The article by N. Foster et al., Processing Pharmaceutical Compounds Using Dense Gas Technology, Ind. Eng. Chem. Res., 2003, 42, 6476-6493, describes that a substance generally exists in one of the three phases: solid, liquid or gas. In a pressure-temperature diagram for a pure substance, each of the phases is well defined and demarcated by phase boundaries. For all molecules, several phases can co-exist until reaching the critical point. As the pressure and temperature increase from the critical point, the liquid and gas phases are indistinguishable. The substance after this point exists as a supercritical fluid.

In the supercritical region, there is only one homogenous medium which has the properties of a liquid and of a gas: the value of the density is similar to that of the liquid, whereas the fluidity properties are characteristic of a gas, so they are therefore referred to as fluids.

In the method of the invention, the compressed fluid can be selected, for example, from the group consisting of carbon dioxide, nitrogen, nitrous oxide, propane, pentane, acetone, diethyl ether, trifluoromethane, chlorodifluoromethane, water, ammonia, and ethanol.

The compressed fluid is preferably carbon dioxide.

In the method of the invention, the inlet pressure of the compressed fluid into the particle formation vessel is greater than the critical pressure thereof and is comprised between 1.01 and 9 times the critical pressure thereof expressed in bars, preferably between 1.1 and 7 times, more preferably between 1.3 and 5 times, and even more preferably between 1.5 and 2.5 times.

Particles presenting the characteristics of the particles prepared with the method of the invention can also be prepared under near-critical conditions.

In the context of the invention, a fluid which is under near-critical conditions relates to a fluid which, for example, is at a temperature greater than the critical temperature thereof and at a pressure slightly below the critical pressure thereof, for example at a pressure 0.9 times the critical pressure thereof, or above the critical pressure thereof, but slightly below the critical temperature thereof, for example 0.9 times the critical temperature thereof expressed in degrees Kelvin.

In the method of the invention, the inlet temperature of the compressed fluid into the particle formation vessel is greater than the critical temperature of said fluid and is comprised between 1.01 and 4 times the critical temperature thereof expressed in degrees Kelvin, preferably between 1.05 and 2.5 times, and more preferably between 1.1 and 1.5 times.

The critical pressure of the carbon dioxide is 73.7 bar, and the critical temperature thereof is 304.1 K.

Therefore, in the case of using carbon dioxide as the compressed fluid in the method of the invention, said fluid is at a pressure comprised between 74.4 and 663.3 bar, preferably between 81 and 515.9 bar, more preferably between 95.8 and 368.5 bar, and even more preferably between 110.5 and 184.2 bar, and at a temperature comprised between 307 and 1216 K, preferably between 319 and 760 K, and more preferably between 334.5 and 456 K.

The compressed fluid is generally miscible or substantially miscible with the fluid which has the solute in solution or in suspension at the point where both fluids come into contact. It is understood as miscible when the two fluids are miscible at any proportion, and substantially miscible relates to the situation in which the fluids can be mixed sufficiently under the operating conditions used and the dissolution of the fluids between them and the precipitation of the solute can be achieved.

The compressed fluid can also possibly contain one or more modifiers, such as water, methanol, ethanol, isopropanol, or acetone, for example.

The modifier is a chemical which, when added to the compressed fluid, modifies the properties thereof, mainly with respect to the solubilizing power.

The amount of the modifying agent, where it is used, is typically below 40% molar with respect to the compressed fluid, preferably below 20% molar, more preferably below 10% molar, and even more preferably it is comprised between 1% and 9% molar.

### Solute Solution

The solute forming part of the solution which is introduced in the particle formation vessel can be ser any substance from which the production of particles is desired. For example, it can be a pharmaceutical active ingredient; pharmaceutically acceptable excipients; colorants or pigments; cosmetic products; food products; plant health products; products for the construction industry; products for the ceramic industry; or products for the paint industry.

The nature of the solute can be organic or inorganic, and it can have a low molecular weight, or it can be a product with a high molecular weight, for example a polymer or a copolymer.

The solute can also be a combination of substances, for example, an active substance and a film-forming substance, or an active substance and a substance capable of forming a matrix.

In a preferred embodiment, the solute is a pharmaceutical active ingredient, a pharmaceutically acceptable excipient, a combination of active ingredients, a combination of excipients, or a mixture thereof.

Said solute is soluble or substantially soluble in the solvent, such that solutions containing at least 0.1% by weight/volume of solute, preferably at least 1% by weight/volume of solute, can be prepared.

The person skilled in the art can select the suitable solvent or solvents to prepare a solution which preferably contains the maximum amount of solute, and thus obtain a higher productivity in preparing the particles, by means of routine experiments.

The solvent can be formed by one or more fluids components, i.e., it can also be formed by a mixture of two or more solvents.

The solute solution can also comprise two or more fluids which are mixed together in the moment of coming into contact with the compressed fluid or immediately before then. Said fluids can be carriers of one or more solutes, such that in the method of the invention they can be combined in solid form when the particles are formed. For example, an active ingredient dispersed in a matrix, or an active ingredient coated by a film-forming substance can be obtained.

In the method of the invention, the solvent can be organic. The following can be mentioned among the organic solvents: methanol, ethanol, isopropanol, acetone, ethyl acetate, acetonitrile, or mixtures thereof.

The solvent can also be aqueous, such that it contains only water, or a mixture of water with an organic solvent miscible therewith, such as methanol, ethanol, isopropanol, or acetone, for example, among others.

### Fluid Inlet

In the method of the invention, the flow of the solute solution in a solvent and the flow of the compressed fluid under supercritical conditions are introduced through separate conduits into the particle formation vessel. Furthermore, the solute solution inlet takes place within the central line of the compressed fluid jet.

At the point where the solute solution encounters the compressed fluid, the angle formed by the axes of each of the flows is comprised between 0° and 180°, preferably between 0° and 100°, preferably between 0° and 95°, and even more preferably between 85° and 95°.

When the angle is 0°, it means that both fluids flow in the same direction, the angle of 90° means that both fluids are flowing in perpendicular directions, and the angle of 180° means that both fluids flow in opposite directions.

The flow rate of the compressed fluid will generally be greater than the flow rate of the solute solution. The ratio between the flow rate of the compressed fluid and the flow rate of the solute solution is typically less than 500, preferably comprised between 5 and 100, and more preferably between 10 and 80.

The flow rate of the compressed fluid is generally selected to achieve an excess thereof with respect to the solvent when both fluids come into contact in the particle formation vessel. For example, the solvent can represent between 1% and 80% molar of the mixture, preferably between 1% and 50% molar, more preferably between 1% and 20% molar, and even more preferably between 1% and 5% molar of the mixture formed when the two fluids come into contact.

The conduit for introducing the solute solution into the particle formation vessel can be made, for example, of stainless steel with an inner diameter comprised between 0.005 and 2.5 mm, preferably between 0.05 and 0.75 mm, and the end part can be tapered and include a nozzle.

The conduit for introducing the compressed fluid into the particle formation vessel can be made of stainless steel with an inner diameter comprised between 0.1 and 3 mm, preferably between 0.2 and 2 mm, and more preferably between 0.4 and 0.8 mm.

The distance between the fluid inlets is a short distance, such as for example a distance comprised between 0 and 50 times the diameter of the outlet of the compressed fluid conduit, preferably between 10 and 40 times, and more preferably between 15 and 25 times.

A suitable distance between both inlets is comprised between 0 and 16 mm, preferably between 2 and 10 mm, and more preferably between 4 and 6 mm.

This means that the solute solution conduit inlet can be located between 0 and 16 mm below the compressed fluid conduit inlet.

The fluid injectors can be coaxial, particularly to allow introducing additional fluids into the particle formation vessel.

The dimensions of the fluid inlets will depend on the scale of the equipment in which the method of the invention is carried out. For example, for industrial scale manufacture, the dimensions can be 10 times greater.

In the case of using carbon dioxide as the supercritical fluid, the following conditions are particularly preferred:
- a temperature comprised between 373 K and 388 K and a pressure comprised between 85 and 105 bar when the diameter of the compressed fluid injector is comprised between 0.5 and 2.5 mm,
- a temperature comprised between 393 K and 403 K and a pressure comprised between 125 and 135 bar when the diameter of the compressed fluid injector is comprised between 350 and 450 microns, and
- a temperature comprised between 408 K and 420 K and a pressure comprised between 195 and 204 bar when the diameter of the compressed fluid injector is comprised between 250 and 349 microns.

### Conditions in the Particle Formation Vessel

In the method of the invention, the temperature in the particle formation vessel is comprised between 0.8 and 1.25 times the critical temperature of the compressed fluid expressed in degrees Kelvin, preferably between 0.96 and 1.12, and more preferably between 1.0 and 1.09.

The pressure in the particle formation vessel is relatively low, as it is comprised between atmospheric pressure and 15 bar, preferably between atmospheric pressure and 10 bar, and more preferably between atmospheric pressure and 5 bar.

In the event that the compressed fluid is carbon dioxide, the temperature in the particle formation vessel is comprised between 243 K and 380 K, preferably between 292 K and 340 K, and more preferably between 304 K and 331 K.

With the method of the invention, a rapid and intimate mixture of the solute solution in a solvent with the compressed fluid is achieved, which allows preparing particles with a small diameter and obtaining a high powder production.

### Description of the Equipment

The method of the invention can be carried out in equipment such as that which is schematically depicted in Figure 1. In the case of a laboratory facility, said equipment comprises the following elements:
- Compressed fluid supply: the compressed fluid is supplied in liquid form from the carboy (1). Said liquid passes through a stainless steel filter with a lumen of 40 microns to eliminate the possible solid residues present therein. The compressed fluid passes through a bath (2) maintained at 258 K, to assure that same is maintained in liquid state until it reaches the pump (3). In the event that the compressed fluid is carbon dioxide, said pump is capable of driving between 3 and 18 kg/h of liquid carbon dioxide at a pressure comprised between 5 and 689 bar. After the pump, the compressed fluid again passes through a stainless steel filter with a lumen of 40 microns to eliminate the possible solid residues present therein, and enters the heat exchanger (4). The thermal liquid present in said exchanger can be heated electrically at a temperature comprised between 303 K and 453 K. The outlet of the heat exchanger is connected to a pipe which conducts the compressed fluid to the particle formation vessel (7).
- Solution supply: the solution containing the product (5) is supplied to an HPLC pump (6) through Teflon tubes having a 10 micron filter made of stainless steel to trap any solid residue present in the solution. The pump is capable of supplying a flow rate comprised between 0.05 and 10 ml/minute at a pressure comprised between 1 and 500 bar. The outlet of the pump conducts the solution to the particle formation vessel through a stainless steel pipe.
- Particle formation vessel: the particle formation vessel is a cylindrical vessel made of stainless steel with an internal volume of 2000 ml, capable of withstanding a pressure of up to 400 bar and a temperature of up to 473 K. The cover of the vessel is attached to the vessel by means of a clamp and it has four openings. The central opening is used for the compressed fluid inlet. The remaining openings are arranged in the form of a T around the central opening. Two of them are used for the product solution inlet, whereas the remaining opening incorporates a thermocouple to measure the temperature inside the vessel. Inside said vessel there is a cylindrical sock-shaped Teflon mesh filter to trap the particles formed.
- Assembly of the injector: the assembly of the compressed fluid injector requires the use of high-pressure stainless steel with a nozzle having a diameter comprised between 0.1 and 3 mm. The solution of the active ingredient is injected into the compressed fluid stream by means of an injector which is outside the compressed fluid nozzle. The solute solution nozzle is made of stainless steel and can have a diameter comprised between 0.005 and 2.5 mm. The injection of the product solution takes place within the central line of the compressed fluid jet, such that the vertical position of the product solution outlet orifice can be modified between 0 and 16 mm from the compressed fluid outlet orifice.
- Outlet valves: the particle formation vessel outlet is connected to the exterior through a valve. After leaving the vessel, the mixture of compressed fluid and the vapors of the solvent, together with the uncollected particles, pass through an additional particle filter before exiting the equipment.

It has surprisingly been observed that in the method of the invention, in which the particles are formed in a vessel at a pressure comprised between atmospheric pressure and 15 bar, particles with suitable characteristics are obtained: small particles with a mean diameter less than the 5 microns, and generally even less than 2 microns, the particles present a limited particle size distribution, and such particles are obtained with low agglomeration.

It has also been surprisingly observed that the method allows preparing products with suitable fluidity from active ingredients and/or excipients with a low glass transition temperature which, under the conditions of the processes described in the state of the art, are plasticized products.

An additional advantage of the method of the invention is the use of a relatively low pressure in the particle formation vessel, facilitating the industrial implementation of the method.

Particles have been prepared with the method of the invention and using the method described in patent application WO-A-03/008082, and also using the aforementioned process referred to as the DELOS process. The complete description of said tests is found in the Example section.

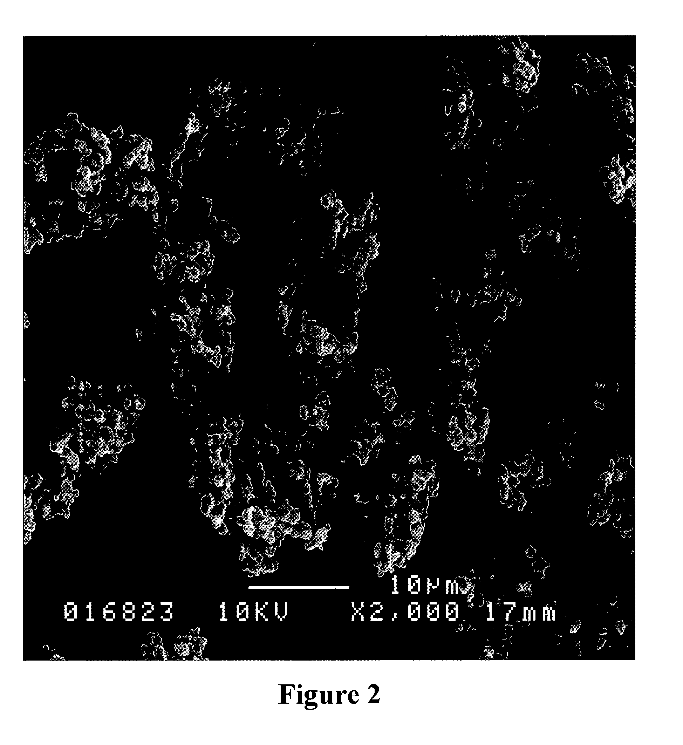

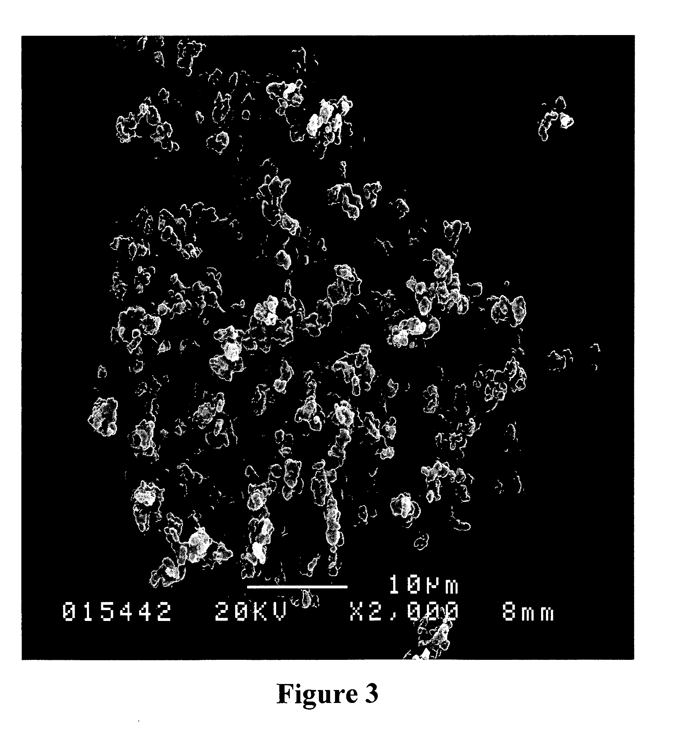

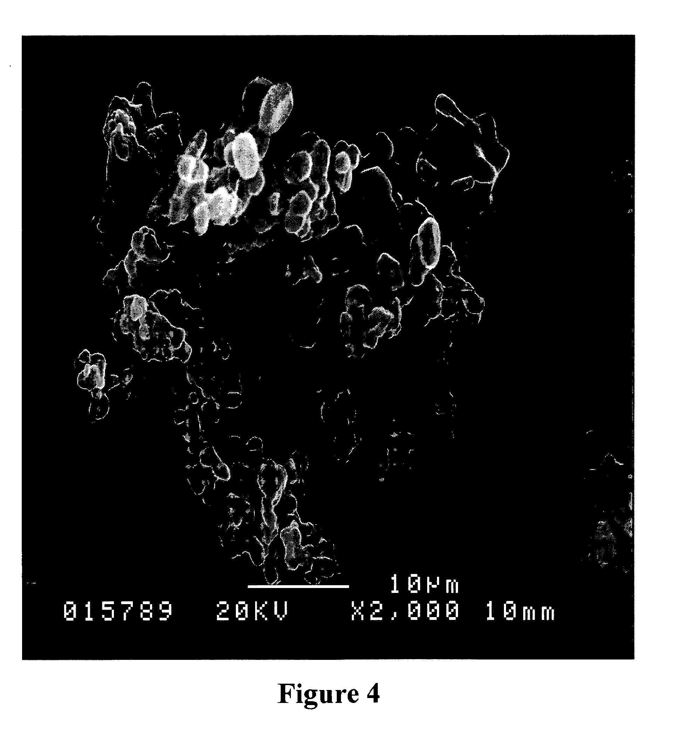

In the case of preparing paracetamol particles, by using the method of the invention with a different pressure in the particle formation vessel, Example 1 and Example 2, non-agglomerated particles are obtained with a mean size of 1.6 microns and 2.2 microns, respectively, and with a width of the size distribution of 1.7 microns (see Figure 2 and Figure 3). It has been observed that by using the method of WO-A-03/008082, strongly agglomerated particles are obtained with a particle size of 13.6 microns and a width of the size distribution of 3.9 microns (see Figure 4).

Particles with suitable fluidity containing PVP and an active ingredient such as nifedipine can be prepared (Example 3) with the method of the invention when a highly plasticizing solute, such as polyvinylpyrrolidone (PVP) of the type referred to as 10K (molecular weight less than 2500), is used.

PVP and nifedipine particles could not be prepared with the method described in patent application WO-A-03/008082 because PVP is a highly plasticizing product which liquefies at moderate pressures of approximately 20 bar.

Nor could particles be prepared with the DELOS process described in Spanish patent application ES-A-2170008 because nifedipine is too insoluble in the carbon dioxide modified according to the mole fraction ranges specified in said process.

The following examples serve to illustrate, not limit, the invention.

### Examples

The following analytical methods have been used to characterize the particles obtained in the examples:

### 1) Scanning electron microscopy

The samples were analyzed in a JEOL JSM6300 apparatus using for that purpose magnifications comprised between x2000 and x10000.

The excitation voltage was comprised between 15 and 20 kV and the working distance between 8 and 10 mm.

The samples were prepared by placing a small amount of powder in a piece of aluminum which had an adhesive layer of lamp black. They were then coated with gold using the EMITECH K550X coater.

### 2) X-ray powder diffraction

Cu-Kα radiation provided by an X'PERT MPD PHILIPS PANALYTICAL 2.1 apparatus equipped with a PW3020 goniometer was used in the X-ray powder diffraction analyses.

The samples were analyzed in a 2θ angle range comprised between 2 and 30 degrees with a pitch of 0.03° and a pitch time of 1 s.

All the samples were subjected to rotation during the measure and were prepared to maximize signal-noise ratio.

### 3) Differential scanning calorimetry

The differential scanning calorimetry (DSC) analyses were carried out in a METTLER TOLEDO DSC 823e apparatus following the manufacturer's instructions.

### 4) Particle size distribution from dry powder

The particle size from dry powder was determined in a MALVERN MASTERSIZER 2000 apparatus coupled to a Sciroco dry powder dispersion apparatus. The dispersion pressure was 2.0 bar with a supply flow of 40%. The particle size curve was analyzed with the computer program of the same apparatus.

The particle size was determined as the mean diameter by volume of the particles. Other parameters such as D₉₀, D₅₀, or D₁₀ were used according to their definition, taking into account that D₉₀ represents the size of 90% of the particles, D₁₀ the size of 10% of the particles, and D₅₀ the size of 50% of the particles. The width of the particle size distribution was calculated from the ratio (D₉₀-D₁₀)/D₅₀.

### 5) Particle size distribution in suspension

The particle size in suspension was determined in a MALVERN ZETASIZER NANO ZS apparatus. The samples were suspended under ultrasonic agitation in a suitable dispersant, for example medium-chain triglycerides manufactured by the company UNIQEMA under the trade name MCT ESTASAN. The suspensions were prepared at 1% by weight/volume, and were subsequently diluted to 0.03% by weight/volume in order to perform the determinations. Polystyrene cuvettes were used, and the acquisition time was 10 seconds. All the determinations were carried out at 293 K. The particle size curve was analyzed with the computer program of the same apparatus.

### Example 1.- Preparing paracetamol particles

18 g of paracetamol were dissolved in 300 ml of acetone, such that a 6% paracetamol solution expressed by weight/volume in acetone was obtained.

The cooling bath was adjusted to 258 K to assure that the compressed fluid, in this case carbon dioxide, reached the HPLC pump in liquid state.

The temperature of the heat exchanger through which the carbon dioxide circulated was set at 408 K to achieve a temperature of 313K inside the particle formation vessel.

The flow of carbon dioxide was set at 12 kg/h, equivalent to 200 ml/minute, and the flow of the paracetamol solution was set at 4 ml/min.

The carbon dioxide outlet orifice and paracetamol solution outlet orifice had a diameter of 400 microns and 250 microns respectively, and the distance between the two openings was 4 mm. The two flows were located perpendicular to one another (90°).

The pressure of the carbon dioxide was 140 bar at the inlet of the particle formation vessel, and the pressure was 1 bar and the temperature was 313 K inside said vessel.

The metering of the paracetamol solution ended after 75 minutes. Then the passage of carbon dioxide was maintained for 5 additional minutes to eliminate traces of residual acetone.

15.7 g of crystalline paracetamol particles, which represented a recovery of 87%, were obtained.

The mean paracetamol particle size was 1.6 microns with a standard deviation of 0.04 microns.

The width of the particle size distribution was 1.7 ± 0.1. This value was obtained from the ratio (D₉₀-D₁₀)/D₅₀,

As can be observed in Figure 2, the particles obtained presented high size uniformity and had a low degree of agglomeration.

When the particles were dispersed at a low pressure (for example 0.2 bar), instead of the standard pressure of 2 bar, the mean particle size increased only to 5.4 microns, which indicated the easy dispersion and the low agglomeration thereof.

### Example 2.- Preparing paracetamol particles

Paracetamol particles were prepared following the process described in Example 1, with the following modifications in the pressure and temperature parameters:
- Pressure of the carbon dioxide at the inlet of the vessel: 135 bar
- Temperature of the carbon dioxide at the inlet of the vessel: 398 K
- Pressure inside the vessel: 10 bar
- Temperature inside the vessel: 308 K.

The two flows were located perpendicular to one another (90°).

15.6 g of crystalline paracetamol particles, which represented a recovery of 87%, were obtained.

The mean particle size was 2.2 microns, and the width of the particle size distribution was 1.7 ± 0.1, calculated according to the formula of Example 1.

As can be observed in Figure 3, the particles presented particle size uniformity and a low agglomeration.

### Comparative Example 1.- Preparing paracetamol particles

In this example paracetamol particles were prepared according to the method described in patent application WO-A-03/008082.

18 g of paracetamol were dissolved in 300 ml of acetone, such that a 6% paracetamol solution expressed by weight/volume in acetone was obtained.

The cooling bath was adjusted to 258 K to assure that the carbon dioxide reached the HPLC pump in liquid state.

The temperature of the heat exchanger through which the carbon dioxide circulated was set at 322 K to achieve a temperature of 313 K inside the vessel where the particles were being formed.

The carbon dioxide flow was set at 12 kg/h, equivalent to 200 ml/minute, and the flow of the paracetamol solution was set at 4 ml/min.

The carbon dioxide outlet orifice and paracetamol solution outlet orifice had a diameter of 400 microns and 250 microns respectively, and the distance between the two openings was 4 mm. The two flows were located perpendicular to one another (90°).

The pressure of the carbon dioxide was 121 bar at the inlet of the particle formation vessel, and the pressure was 95 bar and the temperature was 313 K inside said vessel.

The metering of the paracetamol solution ended after 75 minutes. Then the passage of carbon dioxide was maintained for 20 additional minutes to eliminate traces of residual acetone.

16.0 g of paracetamol, which represented a recovery of 89%, were obtained.

The mean paracetamol particle size was 13.6 microns with a standard deviation of 1.4 microns.

The width of the particle size distribution was 3.9 ± 0.1, calculated in the same way as in Example 1.

As can be observed in Figure 4, the particles obtained were more agglomerated and presented less size uniformity in comparison with the particles obtained with the method of the invention in Example 1.

When the particles were dispersed at a low pressure, such as 0.2 bar, the mean particle size increased to values greater than 30 microns due to the agglomerates that were present in the product.

### Example 3.- Preparing nifedipine and polyvinylpyrrolidone particles

5.0 g of nifedipine and 5.0 g of polyvinylpyrrolidone (10K) were dissolved in 72 ml of a mixture of acetone and methanol (2:1 by volume).

The cooling bath was adjusted to 258 K to assure that the carbon dioxide would reach the HPLC pump in liquid state.

The temperature of the heat exchanger through which the carbon dioxide circulated was set at 398 K to achieve a temperature of 293 K inside the particle formation vessel.

The flow of carbon dioxide was set at 12 kg/h, equivalent to 200 ml/minute, and the flow of the paracetamol solution was set at 4 ml/min.

The carbon dioxide outlet orifice and paracetamol solution outlet orifice had a diameter of 400 microns and 250 microns respectively, and the distance between the two openings was 4 mm. The two flows were located perpendicular to one another (90°).

The pressure of the carbon dioxide was 128 bar at the inlet of the particle formation vessel, and the pressure was 1 bar and the temperature was 293 K inside said vessel.

The metering of the paracetamol solution ended after 18 minutes. Then the passage of carbon dioxide was maintained for 5 additional minutes to eliminate traces of residual acetone.

8.5 g of the mixture of nifedipine and polyvinylpyrrolidone, which represented a recovery of 85%, were obtained.

The mean particle size obtained was 2.7 microns.

The obtained combination of nifedipine and polyvinylpyrrolidone was a solid which had an appearance of fluid powder similar to talc.

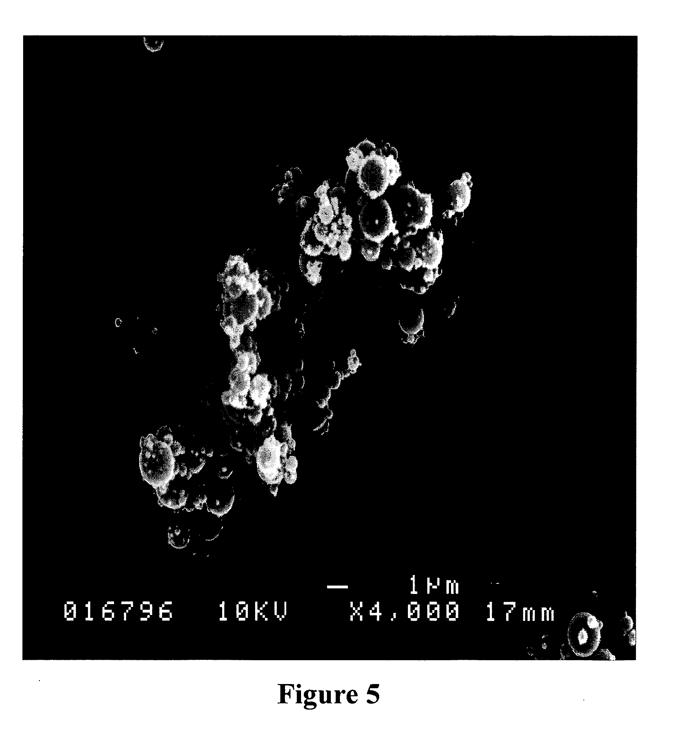

As can be observed in Figure 5, the particles obtained presented high size uniformity and were non-agglomerated.

### Comparative Example 2.- Preparing nifedipine and polyvinylpyrrolidone particles

Nifedipine and polyvinylpyrrolidone particles could not be prepared according to the method described in patent application WO-A-03/008082 because polyvinylpyrrolidone is highly plasticizing and liquefies at moderately high pressures of approximately 20 bar.

### Comparative Example 3.- Preparing nifedipine and polyvinylpyrrolidone particles

Nifedipine and polyvinylpyrrolidone particles could not be prepared according to the DELOS process described in Spanish patent application ES-A-2170008 because nifedipine is too insoluble in the carbon dioxide modified according to the mole fraction ranges specified in said process.

## Claims

1. A method for preparing particles of the invention which comprises introducing in the particle formation vessel through separate conduits:
1) a flow of a compressed fluid under supercritical conditions, and
2) a flow of a solute solution in a solvent,
and collecting the particles formed,
wherein:
i. the inlet pressure of the compressed fluid into the particle formation vessel is comprised between 1.01 and 9 times the critical pressure thereof expressed in bars,
ii. the inlet temperature of the compressed fluid into the particle formation vessel is comprised between 1.01 and 4 times the critical temperature thereof expressed in degrees Kelvin,
iii. the angle formed by the axes of each of the flows is comprised between 0° and 180°,
iv. the inlet of the solute solution takes place within the central line of the compressed fluid jet,
v. the temperature in the particle formation vessel is comprised between 0.8 and 1.25 times the critical temperature of the compressed fluid expressed in degrees Kelvin,
**characterized in that** the particle formation vessel is at a pressure comprised between atmospheric pressure and 15 bar.

2. The method according to claim 1, **characterized in that** the compressed fluid is selected from the group consisting of carbon dioxide, nitrogen, nitrous oxide, propane, pentane, acetone, diethyl ether, trifluoromethane, chlorodifluoromethane, water, ammonia, and ethanol.

3. The method according to claim 2, **characterized in that** the compressed fluid is carbon dioxide.

4. The method according to any of claims 1 to 3, **characterized in that** the inlet pressure of the compressed fluid into the particle formation vessel is comprised between 1.1 and 7 times the critical pressure thereof expressed in bars.

5. The method according to claim 4, **characterized in that** the pressure is comprised between 1.3 and 5 times the critical pressure thereof expressed in bars.

6. The method according to claim 5, **characterized in that** the pressure is comprised between 1.5 and 2.5 times the critical pressure thereof expressed in bars.

7. The method according to any of claims 1 to 6, **characterized in that** the inlet temperature of the compressed fluid into the particle formation vessel is comprised between 1.05 and 2.5 times the critical temperature thereof expressed in degrees Kelvin.

8. The method according to claim 7, **characterized in that** the temperature is comprised between 1.1 and 1.5 times the critical temperature thereof expressed in degrees Kelvin.

9. The method according to any of claims 1 to 8, **characterized in that** the solute is a pharmaceutical active ingredient, a pharmaceutically acceptable excipient, a combination of active ingredients, a combination of excipients, or a mixture thereof.

10. The method according to any of claims 1 to 9, **characterized in that** the angle formed by the axes of each of the flows is comprised between 0° and 100°.

11. The method according to claim 10, **characterized in that** the angle is comprised between 85° and 95°.

12. The method according to any of claims 1 to 11, **characterized in that** the temperature in the particle formation vessel is comprised between 0.96 and 1.12 times the critical temperature of the compressed fluid expressed in degrees Kelvin.

13. The method according to claim 12, **characterized in that** the temperature is comprised between 1.0 and 1.09 times the critical temperature of the compressed fluid expressed in degrees Kelvin.

14. The method according to any of claims 1 to 13, **characterized in that** the pressure in the particle formation vessel is comprised between atmospheric pressure and 10 bar.

15. The method according to claim 14, **characterized in that** the pressure is comprised between atmospheric pressure and 5 bar.
